# EUROPÄISCHE PATENTSCHRIFT

(11) **EP 0 520 237 B1**
(45) Veröffentlichungstag und Bekanntmachung des Hinweises auf die Patenterteilung: **27.01.1999**
(21) Anmeldenummer: 92109712.7
(22) Anmeldetag: 10.06.1992
(51) Int. Cl.: A61L 27/00, A61L 25/00, A61K 38/18

(54) **Knochenersatzmaterial mit Fibroblasten-Wachstums-Faktoren**
Bone replacement material containing fibroblast growth factors
Matériau de prothèse osseuse contenant des facteurs de croissance de fibroblastes

(30) Priorität: 26.06.1991 DE 4121043
(43) Veröffentlichungstag der Anmeldung: 30.12.1992
(73) Patentinhaber: MERCK PATENT GmbH, 64271 Darmstadt (DE)
(72) Erfinder: Nies, Berthold, Dr., W-6105 Ober-Ramstadt (DE); Dingeldein, Elvira, Dr., W-6072 Dreieich (DE); Wahlig, Helmut, Dr., W-6100 Darmstadt (DE)

(56) Entgegenhaltungen:
- EP-A- 0 308 238
- EP-A- 0 345 660
- EP-A- 0 361 896
- EP-A- 0 406 856
- WO-A-89/12464
- WO-A-90/01955
- WO-A-90/03797
- WO-A-90/11366
- GB-A- 2 176 192

## Beschreibung

Die Erfindung betrifft Knochenersatzmaterialien, die in einer porösen Matrix, bestehend im wesentlichen aus Calcium-Mineralien, ein oder mehrere Polypeptide mit der biologischen Wirkung von Fibroblasten-Wachstumsfaktoren enthalten.

Unter Knochenersatzmaterialien sind Materialien zu verstehen, die als Implantate für den Ersatz oder die Rekonstitution von Knochenstrukturen aufgrund von Defekten nach krankheits- oder unfallbedingten operativen Eingriff dienen. Beispielhaft zu nennen sind Implantatformkörper wie Knochenprothesen verschiedenster Art, Knochenverbindungselemente etwa in Form von Markraumnägeln, Knochenschrauben und Osteosyntheseplatten, Implantatmaterialien zur Auffüllung von Spongiosa-Knochendefekten oder von Zahnextraktionshöhlen sowie zur plastischchirurgischen Behandlung von Konturdefekten im Kiefer-Gesichtsbereich.

Für den Einheilungsprozeß werden solche Implantatmaterialien als besonders günstig angesehen, die eine hohe Bioaktivität aufweisen, nämlich dahingehend, daß sie im Organismus angenommen und in ihm integriert werden. Im Falle von Knochenersatzmaterial bedeutet dies, daß es bald mit körpereigenem Gewebe, inbesondere mit dem Knochen, fest und dauerhaft verwachsen soll.

Es ist bekannt, daß bislang die günstigsten Einheilungsresultate praktisch nur mit körpereigenen Materialien, d.h. also mit Knochentransplantaten, erreicht werden. Die Verfügbarkeit von Knochentransplantaten ist naturgemäß begrenzt. Autologe Transplantate, also Transplantate vom selben Individuum sind, sofern überhaupt in geeigneter Form und Menge vorhanden, nur durch mindestens einen zusätzlichen operativen Eingriff entnehmbar, wodurch wiederum ein zusätzlicher Heilungsvorgang am Entnahmeort bedingt wird. Gleiches gilt prinzipiell auch für homologe Transplantate, also Transplantate von Spenderindividuen der gleichen Art. Bei diesen kommen noch Probleme der Verträglichkeit hinzu sowie auch die heute immer noch nicht völlig auszuschließende Infektionsgefahr mit Viren, wie insbesondere Hepatitis- und HIV-Viren. Weiterhin ist die Lagerung von Spendermaterial in Knochenbanken aufwendig und letztendlich zeitlich nur begrenzt.

Aus der WO-A-9001955 ist ein gereinigtes Knochenersatzmaterial für die Medizin bekannt, wobei die einzelnen Partikel des Minerals von allem endogenen organischen Material befreit sind und auf den Oberfläche ein resorbierbares natürliches oder synthetisches makromolekulares Material aufweist.

Implantatmaterialien für den Knochenersatz aus nicht körperverwandten synthetischen oder aus körperverwandten Materialien können, je nach Natur und Beschaffenheit, ein bioinertes bis bioaktives Verhalten zeigen. Die Einheilungsresultate von körpereigenen Knochentransplantaten konnten bislang jedoch noch von keinem synthetischen Implantatmaterial erreicht werden.

Der Erfindung lag daher die Problemstellung zugrunde, ein Knochenersatzmaterial zur Verfügung zu stellen, dessen biologische Aktivität der von körpereigenen Knochentransplantaten möglichst nahe kommt.

Es wurde nun gefunden, daß dies von einem Knochenersatzmaterial erreicht wird, das in einer porösen Matrix, bestehend im wesentlichen aus Calcium-Mineralien, ein oder mehrere Polypeptide mit der biologischen Wirkung von Fibroblasten-Wachstumsfaktoren enthält.

Gegenstand der Erfindung ist daher ein Knochenersatzmaterial, das in einer porösen Matrix, bestehend im wesentlichen aus Calcium-Mineralien, ein oder mehrere Polypeptide mit der biologischen Wirkung von Fibroblasten-Wachstumsfaktoren enthält.

Fibroblasten-Wachstumsfaktoren (Fibroblast Growth Factors, FGF), die zur Klasse der körpereigenen Peptid-Wachstumsfaktoren gehören, wurden usprünglich als Substanzen in Gehirn und Hypophyse nachgewiesen und daraus isoliert und zeigten eine das Wachstum von Fibroblasten fördernde Aktivität. FGFs sind bekannt als wirksame gefäßbildende Faktoren, die u.a. für die Neovaskularisation bei der Wundheilung verantwortlich sind. Nähere Details zu FGFs einschließlich ihrer Abwandlungsprodukte, zu ihrer Isolierung bzw. Herstellung, ihrer Struktur, ihrer biologischen Aktivitäten und deren Mechanismen sowie zu entsprechenden medizinischen Anwendungen können der inzwischen umfangreichen Fachliteratur entnommen werden. Eine umfassende Übersicht bietet beispielsweise A. Baird and P. Böhlen, Fibroblast Growth Factors, in: Peptide Growth Factors and their Receptors I (editors: M.B. Sporn and A.B. Roberts) Springer Verlag Berlin, Heidelberg, New York 1990.

Neben einer Fülle von positiven Wirkungen von FGFs in verschiedensten Indikationsfeldern wurde in neuester Zeit auch in einzelnen Fällen über Einflüsse von FGFs bei der Knochenbildung berichtet, z.B. in Biomaterials 11, 38-40 (1990). In Acta Orthop. Scand. 60, (4) 473-476 (1989) wurde berichtet, daß in Implantaten aus demineralisierter Knochenmatrix (DBM), die mit rekombinantem humanen basischen FGF beladen waren und die Ratten intramuskulär implantiert wurden, ein erhöhter Anteil von mineralisiertem Gewebe gefunden wurde. DBM an sich ist bekannt als Knochenwachstums-fördernde Substanz, da in ihr selbst noch intakte körpereigene Faktoren verschiedenster Art mit Knochenwachstums-fördernder Aktivität enthalten sind. Die biologische Aktivität von DBM ist jedoch, je nach Herkunft und Vorbehandlung, unterschiedlich und in keiner Weise auf ein reproduzierbares Niveau standardisierbar. Darüber hinaus ist DBM als Implantatmaterial für den Knochenersatz aufgrund mangelnder mechanischer Festigkeit praktisch ungeeignet. Aus den publizierten Befunden war in keiner Weise abzuleiten, daß mit dem erfindungsgemäßen Knochenersatzmaterial ein Material zur Verfügung gestellt werden könnte, daß die mechanischen Eigenschaften künstlicher Implantatmaterialien mit der biologischen Aktivität vereint, wie sie nur Knochentransplantate aufweisen.

Die erfindungsgemäßen Knochenersatzmaterialien sind durch das gemeinsame Merkmal gekennzeichnet, daß sie in einer porösen Matrix ein oder mehrere Polypeptide mit der biologischen Wirkung von FGF enthalten. Als erfindungsgemäß geeignete Wachstumsfaktoren sind somit nicht nur die "klassischen" FGFs wie der saure Fibroblasten-Wachstumsfaktor (acidic Fibroblast Growth Factor, aFGF) und der basische Fibroblasten-Wachstumsfaktor (basic Fibroblast Growth Factor, bFGF) anzusehen, sondern alle peptidischen Wachstumfaktoren, die die biologische Wirkung von FGF zeigen.

Zum engeren Kreis von FGFs zählen native FGFs, insbesondere bovinen und humanen Ursprungs sowie rekombinant hergestellte FGFs. Bevorzugt sind insbesondere rekombinant hergestelltes humanes aFGF und bFGF. Näheres zu rekombinant hergestellten bovinen wie humanen aFGFs und bFGFs kann beispielsweise den folgenden Patentdokumenten entnommen werden: EP 228 449, EP 248 819, EP 259 953, EP 275 204. Zum weiteren Kreis von FGFs zählen auch Muteine, die sich von aFGF bzw. bFGF in einem gewissen Umfang in Zahl und/oder Sequenz der Aminosäuren unterscheiden ohne daß hiermit eine wesentliche Wirkungsveränderung verbunden ist. Der weitere Kreis von FGFs umfaßt schließlich noch verwandte Peptide mit zum Teil deutlich verschiedenen Aminosäuresequenzen mit der Wirkung von FGF sowie mit die Wirkung von FGF verstärkender Aktivität. Als Literaturhinweis seien beispielhaft die folgenden Patentdokumente angeführt: EP 148 922, EP 226 181, EP 281 822, EP 288 307, EP 319 052, EP 326 907 und WO 89-12645.

Zu FGFs im Sinne der Erfindung zählen weiterhin Derivate dieser Peptide, die mit stabilisierenden und/oder aktivitätssteigernden Agentien erhalten werden. Es sind dies insbesondere gegen Säure stabilisierte Formen von aFGF und bFGF, die als stäbilisierende Agentien beispielsweise Glykosaminglykane wie Heparin, Heparinfragmente, Heparansulfat und Dermatansulfat oder Glukansulfate wie Dextransulfat und Cyclodextrinsulfat enthalten. FGF-Derivate dieser Art sind beispielsweise beschrieben in EP 251 806, EP 267 015, EP 312 208, EP 345 660, EP 406 856, EP 408 146, WO 89-12464, WO 90-01941 und WO 90-03797.

Besonders bevorzugt für die Anwendung in den erfindungsgemäßen Knochenersatzmaterialien sind Formen von rekombinant hergestelltem humanen bFGF wie sie in EP 248819 beschrieben sind.

In den erfindungsgemäßen Knochenersatzmaterialien können die FGFs in einer Konzentration von 1 ng/cm³ - 1 mg/cm³ vorliegen. Die Wahl der Konzentration innerhalb des genannten Bereichs kann abhängig sein von Art und Form und der Aktivität des im Einzelfall einzusetzenden FGF sowie von der Natur des im Einzelfall vorgesehenen Implantatwerkstoffes und dessen ggf. inhärent vorhandene Bioaktivität. Vorzugsweise liegt die Konzentration an FGF im Bereich zwischen 1 µg/cm³ bis 100µg/cm³.

In den erfindungsgemäßen Knochenersatzmaterialien können grundsätzlich alle bekannten und üblichen Implantatwerkstoffe vorliegen, sofern diese eine poröse Matrix zur Aufnahme von FGF darstellen oder aufweisen. Implantatwerkstoffe können in die Klassen mineralische, insbesondere keramische Werkstoffe, physiologisch akzeptable metallische Werkstoffe, physiologisch akzeptable Polymerwerkstoffe und Verbundwerkstoffe aus zwei oder mehr Materialien der genannten Art eingeteilt werden. Diese Werkstoffe können als Ganzes eine poröse Matrix bilden, etwa in Form von porösen Implantantformkörpern oder es können nur bestimmte Anteile des Werkstoffs als poröses Material vorliegen bzw. bestimmte Bereiche eines Implantatformkörpers eine poröse Matrix darstellen. Die letzten beiden Fälle können beispielsweise in der Form realisiert sein, daß ein Verbundwerkstoff oder ein Knochenzement eine poröse Komponente enthält bzw. ein Implantat mit einer porösen Oberflächenbeschichtung oder einer entsprechend aufgerauhten Oberfläche versehen ist.

Für die erfindungsgemäßen Knochenersatzmaterialien sind von der Werkstoffseite Materialien bevorzugt, die mineralischer und insbesondere keramischer Natur sind. Ein vorteilhafter Aspekt der Erfindung ist, daß an sich bioinerte Materialien, wie etwa oxidkeramische Werkstoffe, durch die Beladung mit FGF biologisch aktiviert werden können und so ein signifikant besseres Einwachs- und Einheilungsverhalten zeigen.

Bevorzugte mineralische Werkstoffe sind allerdings solche, die an sich bioaktiv sind. Dies trifft vornehmlich auf Materialien zu, die auf Calcium-haltigen Materialien basieren, wie inbesondere Calciumcarbonat, Calciumphosphate und von diesen Verbindungen abgeleitete Systeme. Aus der Gruppe der Calciumphosphate sind als bevorzugt Hydroxylapatit, Tricalciumphosphat und Tetracalciumphosphat zu nennen.

Implantatwerkstoffe auf mineralischer Basis gewährleisten jedoch meist nur dann eine hohe mechanische Stabilität, wenn sie als Keramiken, d.h. also in Form von bei ausreichend hohen Temperaturen gesinterten Materialien bzw. Werkstücken eingesetzt werden.

Knochenersatzmaterial auf Basis von Calciumphosphat-Keramiken gelten aufgrund ihrer chemischen Verwandtschaft mit der Mineralphase natürlicher Knochen als bioaktiv. Natürlicher Knochen besteht in seiner Mineralphase überwiegend aus Hydroxylapatit, einem Calciumphosphat der Summenformel Ca₅(PO₄)₃OH.

Hydroxylapatit synthetischen oder organischen Ursprungs, etwa aus natürlichem Knochenmaterial, ist daher ein häufig verwendeter Rohstoff zur Herstellung von Implantaten für den Knochenersatz. Hydroxylapatit-Keramik ist im Organismus im wesentlichen nicht resorbierbar. Das heißt, das körperfremde Material bleibt über lange Zeit praktisch unverändert erhalten und die Integration in den Organismus erfolgt im wesentlichen durch Verwachsen mit vorhandenem und sich neu bildenden Knochen und Einwachsen im umgebenden Gewebe.

Tricalciumphosphat ist unter bestimmten Umständen im Organismus resorbierbar. Tetracalciumphosphat ist im wesentlichen nicht bioresorbierbar.

Ein besonders günstiges Einwachsverhalten zeigen poröse Calciumphosphat-Keramiken. Besonders bevorzugt sind hierbei Materialien basierend auf natürlichem Knochen, der durch verschiedene Behandlungen mineralisiert und in ein keramisches System überführt wird, wobei die Struktur des Knochens möglichst erhalten bleiben soll. Den Verfahren gemeinsam ist die Entfernung der organischen Knochenbestandteile und die anschließende Verfestigung zur Keramik durch Sinterung bei entsprechenden Temperaturen. Die Entfernung der organischen Anteile erfolgt durch chemische Lösungsvorgänge oder durch pyrolytische Verfahren.

Näheres zu Knochenkeramiken und besonders günstige Verfahren zu ihrer Herstellung kann beispielsweise den Patentdokumenten DE 37 27 606, DE 39 03 695, DE 41 00 897 und DE 40 28 683 entnommen werden.

Knochenkeramikimplantate zeigen aufgrund ihrer ausgezeichneten Übereinstimmung mit dem Porensystem natürlichen Knochens erhebliche biologische Vorteile beim Einwachsverhalten und der Heilung im Organismus. Besonders bevorzugt ist SpongiosaKnochenkeramik aufgrund seiner hochporösen, dreidimensional offenporigen Netzwerkstruktur.

Formkörper aus keramischem Material, insbesondere der vorgenannten Art, werden in erster Linie für den Ersatz von tragenden Knochenstrukturen eingesetzt, die hohen mechanischen Belastungen Stand halten müssen. Beispiele hierfür sind Knochenprothesen und Knochenverbindungselemente wie etwa Markraumnägel, Knochenschrauben und Osteosyntheseplatten.

Genauere klinische Untersuchungen haben gezeigt, daß offen vorliegende mineralische Kontaktflächen in Implantaten aus Calciumphosphat-Keramik bevorzugt die Neubildung von mineralischer Knochenmatrix stimulieren, wodurch sich eine feste Verwachsung des Implantats ergibt. Gefördert wird dies weiter noch bei porösen Implantaten, wo sich aufgrund der höheren Oberfläche und durch Einsprossung von neuem Knochengewebe eine besonders intensiv verzahnte und damit mechanisch stabile Verwachsung ausbildet. Bei Implantatmaterialien aus überwiegend polymeren Werkstoffen oder aus bioinerten Materialien bildet sich statt dessen zunächst bevorzugt im Kontaktbereich Bindegewebe, was zu einer nur mäßig festen Verwachsung führt.

Es hat sich nun gezeigt, daß die erfindungsgemäßen Knochenersatzmaterialien, im wesentlichen unabhängig von der Art des Werkstoffes, durch die Beladung mit FGF nach der Implantation im Kontaktbereich und, je nach dem ob sie aufgrund Porosität und/oder Resorption durchwachsbar sind, auch in ihrem Inneren eine erhebliche Neubildung von mineralischer Knochenmatrix stimulieren. Dies ist in jedem Fall signifikant höher als in entsprechenden unbeladenen Implantaten. Hierbei konnte bei mit FGF beladen porösen Implantaten auf Basis von Calcium-Mineralien, insbesondere Calciumphosphat-Keramiken, ein ausgeprägter synergistischer Effekt beobachtet werden. So zeigte sich in präklinischen Modellversuchen bei mit FGF beladenen Knochenkeramik-Implantaten sechs Wochen nach der Implantation eine vollständige Inkorporation in den Knochen durch Ein- und Durchwachsung mit neugebildeter, überwiegend mineralisierter Knochenmatrix. Ein vergleichbares Ergebnis wurde nur von autologen Knochentransplantaten erreicht, während beispielsweise bei unbeladener Knochenkeramik und bei DBM nur in den Kontaktbereichen zum vorliegenden Knochen eine Verwachsung durch Neubildung von Knochenmatrix festgestellt werden konnte. Es wird angenommen, daß sich die knochenwachstumsfördernde Wirkung von FGF und die Bioaktivität von Calcium-haltigen Implantatwerkstoffen, wie insbesondere Knochenkeramik, sich gegenseitig verstärken und so zu einer beschleunigten Einheilung und Inkorporation des Implantates führen.

Der positive Einfluß von FGF auf das Einheilungsverhalten von Implantaten für den Knochenersatz ist, wie schon erwähnt, auf praktisch alle Arten von Knochenersatzmaterialien und Implantatwerkstoffen übertragbar, sofern diese so geartet bzw. gestaltet sind, daß sie eine poröse Matrix zur Aufnahme von FGF und Wiederabgäbe an den Organismus aufweisen, zweckmäßigerweise zumindest vornehmlich im Kontaktbereich mit dem Körpergewebe. Diese Voraussetzungen erfüllen beispielsweise auch Implantate aus metallischen Werkstoffen, die in sich porös sind oder eine poröse Oberflächenbeschichtung, vorzugsweise aus dem bioaktiven Hydroxylapatit, oder die eine porös strukturierte oder zumindest aufgerauhte Oberfläche aufweisen. Gleiches gilt für Implantate aus polymeren Werkstoffen, anderen Keramikmaterialien oder aus Verbundwerkstoffen.

Grundsätzlich können die erfindungsgemäßen Knochenersatzmaterialien nicht nur als Implantatformkörper vorliegen, sondern auch in Pulver- oder Granulatform, je nachdem wie es der Einsatzort und der Anwendungszweck erfordert.

Als Verbundmaterialien kommen vorzugsweise solche in Betracht, bei denen zumindest eine Komponente als poröse Matrix zur Aufnahme von FGF vorliegt. Zweckmäßig sind entsprechende Knochenersatzmaterialien auf Basis von Verbundwerkstoffen, in denen eine poröse mineralische Matrix in Pulver- oder Granulatform vorliegt und im Verbund mit einem physiologisch akzeptablen polymeren Werkstoff einen Formkörper bildet. Verbundmaterialien dieser Art können der einschlägigen Fachliteratur entnommen werden, beispielsweise den Patentdokumenten WO 90-01342 und WO 90-01955, in denen Implantatwerkstoffe auf Basis von Calciumphosphat- bzw. Knochenkeramikpartikeln und bioresorbierbarem Polymer beschrieben sind.

In analoger Weise kann auch die Bioaktivität von Knochenzementen gesteigert werden. Knochenzemente bestehen überwiegend aus Acrylatsystemen, die mineralische Füllstoffe, meist auf Basis von Calciumverbindungen, enthalten. Erfindungsgemäß kann beispielsweise mit FGF beladenes poröses Hydroxylapatitpulver bzw. -granulat als Füllstoffkomponente in Knochenzement eingesetzt werden.

Die Herstellung der erfindungsgemäßen Knochenersatzmaterialien durch Beladung der jeweiligen porösen Matrix mit Polypeptiden mit der Wirkung von FGF ist an sich problemlos. Zweckmäßigerweise geht man von einer geeigneten flüssigen oder halbflüssigen Präparation von FGF, beispielsweise in Form einer gepufferten wäßrigen Lösung, einer Suspension oder eines Gels, aus und läßt diese in der vorgesehenen Dosierungsmenge in die poröse Matrix des Knochenersatzmaterials vollständig einziehen. Damit, bzw. nach einer ggf. erforderlichen Trocknung, ist das Knochenersatzmaterial bereits einsetzbar oder nach den für derartige Materialien für die medizinische Anwendung erforderlichen Vorsichtsmaßnahmen, lagerbar. Auf diese Weise sind poröse Implantatformkörper, vorzugsweise aus Knochenkeramik, mit poröser Oberfläche versehene Implantate und poröse partikelförmige Komponenten für Verbundwerkstoffe und Knochenzemente mit FGF beladbar.

In einer bevorzugten Ausführungsform liegt das erfindungsgemäße Knochenersatzmaterial in Form eines gebrauchsfertigen Implantationssets aus zwei oder mehr getrennten Komponenten vor, worin eine Komponente die poröse Matrix und eine andere Komponente eine Lösung des Polypeptids mit der Wirkung von FGF beinhaltet. Eine derartige Ausführungsform ist besonders zweckmäßig, um mögliche Stabilitätsprobleme, die bei einer Langzeitlagerung von bereits fertig konfektionierten erfindungsgemäßen Knochenersatzmaterialien auftreten könnten, wirksam zu begegnen. So wurde beispielsweise in der Fachliteratur berichtet, daß Calciumionen, die ja in den hier bevorzugten Werkstoffen vorliegen, einen destabilisierenden Einfluß auf FGF ausüben können. Die Anwendung der erfindungsgemäßen Knochenersatzmaterialien in Form eines derartigen Implatationssets erfolgt in der Weise, daß man kurz, vor oder während des chirurgischen Eingriffs für die Implantation die poröse Matrix des jeweiligen Implantatwerkstoffes mit der FGF-enthaltenden Lösung in der vorbeschriebenen Weise belädt. Besonders zweckmäßig ist eine derartige Ausführungsform für den Fall, daß die poröse Matrix von einem Implantatformkörper selbst gebildet wird, wobei als Werkstoff in erster Linie mineralische, vorzugsweise keramische Werkstoffe und insbesondere gesinterte Knochenkeramik in Betracht kommen.

Je nach Ausführungsform stellt somit das erfindungsgemäße Knochenersatzmaterial einen zumindest gleichwertigen Ersatz für autologe und homologe Knochentransplantate dar oder ist für andere Formen des Knochenersatzes eine erhebliche Verbesserung in Bezug auf das Einheilungsverhalten.

### Beispiel 1

### Herstellung von Implantatformkörpern

Aus Spongiosa-Hydroxylapatit-Knochenkeramikrohlingen, hergestellt nach DE 40 28 683, werden mittels Diamantfräse zylindrische Formkörper von 10,00 mm Höhe und 9,55 mm Durchmesser gefertigt.

Ein Teil dieser Formkörper wird jeweils mit 100 µl einer Lösung, enthalted 50 µg rekombinant hergestelltes humanes bFGF imprägniert, getrocknet und bei 4-6 °C bis zum Implantationszeitpunkt gelagert.

Die übrigen Formkörper dienen Vergleichszwecken.

### Beispiel 2

### Vergleichende tierexperimentelle Untersuchung

- **Tierart:**: Minischwein, adult, weiblich, 6 Gruppen, 8 Implantate pro Gruppe
- **Implantate:**: a) Spongiosa-Hydroxylapatit-Keramik mit FGF (gemäß Beispiel 1)
b) Spongiosa-Hydroxylapatit-Keramik
c) DBM
d) Spongiosa-Hydroxylapatit-Keramik, imprägniert mit DBM
e) autologes Spongiosatransplantat, paßgenau mit Zwillingsfräse entnommen.
f) homologes Spongiosatransplantat, paßgenau mit Zwillingsfräse entnommen, Lagerung bis zum Implantationszeitpunkt bei -30 °C
- **Implantationsort:**: In das Patellagleitlager der Femurkondyle, linksseitig und rechtsseitig

Nach 6 Wochen werden die Knochen operativ entnommen und durch histologische Untersuchung die Knochenneubildung und die Mineralisation erfaßt.

### Ergebnis:

### a) Spongiosa-Hydroxylaptitkeramik mit FGF

Knochenneubildung vom Knochenbett bis in das Zentrum des Implantats; vollständige Inkorporation

### b) Spongiosa-Hydroxylapatitkeramik

Marginaler knöcherner Kontakt mit dem Implantat; Einwachsen nur in den Randbereich des Implantats

### c) DBM

Marginaler knöcherner Kontakt mit dem Implantat; Einwachsen nur in den Randbereich des Implantats

### d) Spongiosa-Hydroxylapatitkeramik, imprägniert mit DBM

Knochenneubildung im Kontaktbereich von Knochenbett und Implantat; amorphes DBM liegt noch vor.

### e) Autologes Spongiosatransplantat

Knochenneubildung vom Knochenbett bis in das Zentrum des Implantats; vollständige Inkorporation.

### f) Homologes Spongiosatransplantat

Knochenneubildung vom Knochenbett, etwa 1/3 bis 1/2 des Implantats erfassend; teilweise Inkorporation.

### Beispiel 3

### Implantationsset

Poröse Spongiosa-Hydroxylapatit-Knochenkeramikformkörper (nach Beispiel 1; unbeladen) werden in entsprechend gestaltete Tiefzieh-Verpackungsformteile gesetzt, deren Kammern exakt den Abmessungen (nur geringes Restvolumen) der Formkörper entsprechen. Die Tiefziehteile werden eingesiegelt sterilisiert und mit einer Umverpackung umgeben.

bFGF-Lösung wird in Citrat-Puffer (10 mMol; pH 5,0) und nach Zugabe von Saccharose-Lösung ( 9 %) gefriergetrocknet und in Ampullen abgefüllt. Dabei werden Ampullenbefüllung und Ampullenvolumen so abgestimmt, daß die spätere Beladung der Keramikformkörper 50 µg bFGF/cm³ Blockvolumen entspricht.

Implantatformkörper-Packungen und bFGF-Ampullen bilden Packungseinheiten als Implantationssets.

### Konditionierung am Operationstisch

Die bFGF-Lösung wird in Citrat-Puffer (pH 5,0) rekonstituiert und anschließend auf eine sterile Spritze aufgezogen.

Nach Öffnen der Umverpackung wird die bFGF-Lösung durch die sterile Innenverpackung in das Tiefziehbehältnis des Keramik-Formkörpers injiziert. Das Injektionsvolumen wird so bemessen, daß der Formkörper vollständig in die bFGF-Lösung eintaucht. Überschüssige bFGF-Lösung wird nach ca. 1 Minute in die Spritze zurückgesaugt. Der Keramik-Formkörper hält etwa so viel Lösung fest, wie seinem Porenvolumen entspricht.

Der beladene Formkörper kann nach Öffnen der Primärverpackung implantiert werden.

## Patentansprüche

1. Knochenersatzmaterial, dadurch gekennzeichnet, daß es in einer porösen Matrix, bestehend im wesentlichen aus Calcium-Mineralien, ein oder mehrere Polypeptide mit der biologischen Wirkung Von Fibroblasten-Wachstumsfaktoren enthält.

2. Knochenersatzmaterial nach Anspruch 1, dadurch gekennzeichnet, daß es den sauren oder basischen Fibroblasten-Wachstumsfaktor enthält.

3. Knochenersatzmaterial nach Anspruch 2, dadurch gekennzeichnet, daß es rekombinant hergestellten Fibroblasten-Wachstumsfaktor enthält.

4. Knochenersatzmaterial nach einem der Ansprüche 2 oder 3, dadurch gekennzeichnet, daß es Muteine oder säurestabilisierte Fibroblastenwachstumsfaktoren enthält.

5. Knochenersatzmaterial nach einem der Ansprüche 1 bis 4, dadurch gekennzeichnet, daß es 1 ng/cm³ bis 1 mg/cm³, Vorzugsweise 1 bis 100 µg/cm³, an Polypeptid enthält.

6. Knochenersatzmaterial nach einem der Ansprüche 1 bis 5, dadurch gekennzeichnet, daß die poröse calcium-mineralische Matrix im wesentlichen aus Calciumphosphat besteht.

7. Knochenersatzmaterial nach Anspruch 6, dadurch gekennzeichnet, daß die poröse calcium-mineralische Matrix aus einer oder mehreren Verbindungen der Gruppe Hydroxylapatit, Tricalciumphosphat, Tetracalciumphosphat besteht.

8. Knochenersatzmaterial nach einem der Ansprüche 1 bis 7, dadurch gekennzeichnet, daß die Calcium-Mineralien aus natürlichem Knochen gewonnen sind.

9. Knochenersatzmaterial nach einem der Ansprüche 1 bis 8, dadurch gekennzeichnet, daß es sich bei der porösen mineralischen Matrix um gesinterte Calciumphosphatkeramik handelt.

10. Knochenersatzmaterial nach Anspruch 8, dadurch gekennzeichnet, daß die poröse mineralische Matrix aus gesinterter Spongiosa-Knochenkeramik besteht.

11. Knochenersatzmaterial nach einem der Ansprüche 1 bis 10, dadurch gekennzeichnet, daß die poröse Matrix als Implantatformkörper Vorliegt oder die Oberfläche bzw. eine Oberflächenbeschichtung desselben bildet.

12. Knochenersatzmaterial nach einem der Ansprüche 1 bis 11, dadurch gekennzeichnet, daß die poröse Matrix in Pulver- oder Granulatform vorliegt.

13. Implantationsset auf Basis eines Knochenersatzmaterials gemäß den Ansprüchen 1 bis 12, dadurch gekennzeichnet, daß es im wesentlichen aus zwei oder mehr getrennten Komponenten besteht, dessen eine Komponente die calcium-mineralisch poröse Matrix und eine andere Komponente eine Lösung oder Suspension des Polypeptids beinhaltet.

14. Implantationsset nach Anspruch 13, dadurch gekennzeichnet, daß die Komponente, die die poröse Matrix bildet, ein Implantatformkörper ist.

15. Implantationsset nach Anspruch 14, dadurch gekennzeichnet, daß der Implantatformkörper aus gesinterter Knochenkeramik besteht.

## Claims

1. Bone replacement material, characterised in that it comprises one or more polypeptides having the biological action of fibroblast growth factors in a porous matrix essentially consisting of calcium minerals.

2. Bone replacement material according to Claim 1, characterised in that it comprises acidic or basic fibroblast growth factor.

3. Bone replacement material according to Claim 2 characterised in that it comprises fibroblast growth factor prepared by a recombinant method.

4. Bone replacement material according to one of Claims 2 or 3, characterised in that it comprises muteins or acid-stabilised fibroblast growth factors.

5. Bone replacement material according to one of Claims 1 to 4, characterised in that it comprises 1 ng/cm³ to 1 mg/cm³, preferably 1 to 100 µg/cm³, of polypeptide.

6. Bone replacement material according to one of Claims 1 to 5, characterised in that the porous caclium-mineral matrix essentially consists of calcium phosphate.

7. Bone replacement material according to Claim 6, characterised in that the porous calcium-mineral matrix consists of one or more compounds from the group consisting of hydroxyapatite, tricalcium phosphate and tetracalcium phosphate.

8. Bone replacement material according to one of Claims 1 to 7, characterised in that the calcium minerals are obtained from natural bone.

9. Bone replacement material according to one of Claims 1 to 8, characterised in that the porous mineral matrix is sintered calcium phosphate ceramic.

10. Bone replacement material according to Claim 8, characterised in that the porous mineral matrix consists of sintered spongiosa bone ceramic.

11. Bone replacement material according to one of Claims 1 to 10, characterised in that the porous matrix is present as a shaped implant article or forms the surface or a surface coating of the same.

12. Bone replacement material according to one of Claims 1 to 11, characterised in that the porous matrix is present in powder or granule form.

13. Implantation set based on a bone replacement material according to Claims 1 to 12, characterised in that it consists of two or more separate components, one component of which comprises the calcium-mineral porous matrix and another component of which comprises a solution or suspension of the polypeptide.

14. Implantation set according to Claim 13, characterised in that the component which forms the porous matrix is a shaped implant article.

15. Implantation set according to Claim 14, characterised in that the shaped implant article consists of sintered bone ceramic.

## Revendications

1. Matériau succédané d'os, caractérisé en ce qu'il contient, dans une matrice poreuse constituée pour l'essentiel de minéraux de calcium, un ou plusieurs polypeptides ayant l'action biologique de facteurs de croissance des fibroblastes.

2. Matériau succédané d'os selon la revendication 1, caractérisé en ce qu'il contient le facteur de croissance des fibroblastes acide ou basique.

3. Matériau succédané d'os selon la revendication 2, caractérisé en ce qu'il contient le facteur de croissance des fibroblastes produit de façon recombinante.

4. Matériau succédané d'os selon l'une des revendications 2 ou 3, caractérisé en ce qu'il contient des mutéines ou des facteurs de croissance des fibroblastes stabilisés vis-à-vis des acides.

5. Matériau succédané d'os selon l'une des revendications 1 à 4, caractérisé en ce qu'il contient de 1 ng/cm³ à 1 mg/cm³, de préférence de 1 à 100 µg/cm³, de polypeptide.

6. Matériau succédané d'os selon l'une des revendications 1 à 5, caractérisé en ce que la matrice poreuse de minéraux de calcium se compose essentiellement de phosphate de calcium.

7. Matériau succédané d'os selon la revendication 6, caractérisé en ce que la matrice poreuse de minéraux de calcium se compose d'un ou plusieurs composés du groupe hydroxylapatite, phosphate tricalcique, phosphate tétracalcique.

8. Matériau succédané d'os selon l'une des revendications 1 à 7, caractérisé en ce que les minéraux de calcium sont obtenus à partir d'os naturels.

9. Matériau succédané d'os selon l'une des revendications 1 à 8, caractérisé en ce que, pour la matrice minérale poreuse, il s'agit d'une céramique de phosphate de calcium frittée.

10. Matériau succédané d'os selon la revendication 8, caractérisé en ce que la matrice minérale poreuse se compose d'une céramique d'os spongieux frittée.

11. Matériau succédané d'os selon l'une des revendications 1 à 10, caractérisé en ce que la matrice spongieuse se présente sous forme de corps moulé en implant ou en forme la surface ou selon les cas un revêtement de surface.

12. Matériau succédané d'os selon l'une des revendications 1 à 11, caractérisé en ce que la matrice poreuse se présente sous forme de poudre ou de granulé.

13. Ensemble pour implantation à base d'un matériau succédané d'os selon les revendications 1 à 12, caractérisé en ce qu'il se compose pour l'essentiel de deux ou plusieurs composants séparés dont l'un contient la matrice poreuse à minéral calcique et un autre contient une solution ou suspension du polypeptide.

14. Ensemble pour implantation selon la revendication 13, caractérisé en ce que le composant qui forme la matrice poreuse est un corps moulé en implant.

15. Ensemble pour implantation selon la revendication 14, caractérisé en ce que le corps moulé en implant se compose de céramique osseuse frittée.
